# EUROPEAN PATENT SPECIFICATION

(11) **EP 3 636 756 B1**
(45) Date of publication and mention of the grant of the patent: **16.02.2022**
(21) Application number: 18908351.2
(22) Date of filing: 20.04.2018
(51) Int. Cl.: C12N 15/10

(54) **METHOD FOR PURIFYING TOTAL MRNA FROM TOTAL RNA USING SLFN13**
VERFAHREN ZUR REINIGUNG VON GESAMT-MRNA AUS GESAMT-RNA UNTER VERWENDUNG VON SLFN13
PROCÉDÉ DE PURIFICATION D'ARNM TOTAL À PARTIR D'ARN TOTAL FAISANT APPEL À SLFN13

(30) Priority: 06.03.2018 CN 201810181007
(43) Date of publication of application: 15.04.2020
(73) Proprietor: Sun Yat-Sen University Cancer Center (SYSUCC), Yuexiu District Guangzhou Guangdong 510060 (CN)
(72) Inventor: GAO, Song, Guangzhou, Guangdong 510060 (CN); YANG, Jinyu, Guangzhou, Guangdong 510060 (CN); XIE, Wei, Guangzhou, Guangdong 510006 (CN); DENG, Xiangyu, Dongguan City, Guangdong Province 523871 (CN)
(74) Representative: Wang, Bo
(86) International application number: PCT/CN2018/083874
(87) International publication number: WO 2019/169701

(56) References cited:
- EP-A1- 0 314 202
- WO-A1-01/25418
- WO-A1-2015/164773
- CN-A- 103 038 338
- WANG ET AL: "Rapid high-yield mRNA extraction for reverse-transcription PCR", JOURNAL OF BIOCHEMICAL AND BIOPHYSICAL METHODS, AMSTERDAM, NL, vol. 70, no. 3, 8 March 2007 (2007-03-08), pages 507-509, XP005917465, ISSN: 0165-022X, DOI: 10.1016/J.JBBM.2006.10.003
- FURAO LIU ET AL: "The Schlafen family: complex roles in different cell types and virus replication : The characteristics and functions of Schlafen family", CELL BIOLOGY INTERNATIONAL., vol. 42, no. 1, 13 June 2017 (2017-06-13), pages 2-8, XP055688375, GB ISSN: 1065-6995, DOI: 10.1002/cbin.10778
- JIN-YU YANG et al.: "Structure of Schlafen 13 reveals a new class of tRNA/rRNA-ta- rgeting RNase engaged in translational control", Nature Communications, vol. 9, no. 1, 21 March 2018 (2018-03-21), XP055639569, ISSN: 2041-1723
- JING, Guozhong et al.: "Purifying Specific mRNA by using immune technology", Progress in Biochemistry and Biophysics, no. 6, 27 December 1979 (1979-12-27), XP009518209, ISSN: 1000-3282
- DATABASE Nucleotide 11 February 2009 (2009-02-11), Strausberg: "Homo sapiens schlafen family member 13, mRNA (cDNA clone MGC:168235 IMAGE:9020612), complete cds", XP055639575, retrieved from NCBI Database accession no. BC136622.1

## Description

### FIELD OF THE INVENTION

The present disclosure belongs to the field of biotechnology, and particularly relates to a method for purifying total mRNA from total RNA with SLFN13 (Schlafen13).

### BACKGROUND OF THE INVENTION

Messenger RNAs (mRNAs) are essential macromolecules of all organisms. Transcription of mRNA is an indispensible stage during the expression of a gene. mRNAs pass the genetic information stored in DNAs to the cellular translational machinery to faithfully produce various proteins that carry out various biological functions. Therefore, mRNAs can reflect the transcription and expression information of a specific cell or tissue at a certain functional state, and is closely related to cell property, growth situation and the like. High-throughput sequencing of transcriptome mRNA is a highly efficient method that is widely used at present for research and healthcare. By acquiring complete sequence information of mRNAs within a single run, the high-throughput mRNA sequencing method can analyze comprehensive transcriptome information such as gene expression, single nucleotide polymorphism (SNP), new transcripts, new isomers, splicing sites, specific expression of alleles and rare transcription. The first important step during a sequencing experiment is to extract total RNA of target cells or tissues, and obtain, as much as possible, high-quality total mRNA with good integrity and high purity. The high-quality mRNA preparation is the prerequisite for the efficiency of subsequent full cDNA library construction, which is realized through a reverse transcription process before accurate and reliable sequencing results can be obtained. In the total RNA extract of an organism, however, mRNAs typically take up only 1% to 5%, whereas 75% to 85% are ribosome (r)RNAs and 10% to 16% are transfer (t)RNAs. In addition, mRNA molecules are highly inhomogenous in terms of molecular weight and abundance. Therefore, to purify high-quality mRNA from total RNA while ensuring the integrity is an indispensible but a difficult step for building a cDNA library. At present, there are mainly two approaches to realize the relative purification of mRNA: one utilizes the fact that most mRNA molecules possess poly(A) sequence at their 3'-termini, and designs a poly dT-containing matrix specifically bound to poly(A), so as to separate poly(A)-containing mRNA from total RNA; the other designs a matrix capable of being specifically bound to a conserved region in rRNA, so as to remove as much as possible rRNA which is the most abundant impurity, of total RNA, and to obtain relatively purified mRNA.

RNA is relatively unstable. It is prone to degradation *in vitro,* especially when exposed to air. The two purification methods above have relatively complicated processes and are difficult to be finished in short time, which greatly increases the risk for RNA degradation. The degradation of RNA can seriously affect the quality of a library, which not only leads to loss of important information, but also introduces many mistakes and errors. More importantly, the purification effect of both the above two methods are not quite ideal. For the first method, it can only ensure 40% to 70% integrity of purified mRNA, which would affect the accuracy of sequencing data and differential display between data sets. For the second method, though the resulted mRNA integrity is better than that of the first method, the final purity of mRNA is much lower. This is because the second method is impotent to remove tRNA, whose amount greatly exceeds mRNA even after rRNA is removed from the total RNA. Therefore, after the final step of the second method, the resulting RNA pool often contains only less than 30% mRNA that is wanted.

Today, the high-throughput RNA sequencing methods are still developing, and the population of the users continues growing. As the integrity and purity of total mRNA extracted from various samples directly determines the quality of the subsequently cDNA library construction, hence the accuracy of the sequencing data, the quality of the mRNA sample becomes a limiting factor that affects the usage, development, and data interpretation of the high-throughput RNA sequencing method. Therefore, it is urgent to develop novel mRNA purification methods (i.e. time-saving, easy-handling, low-costs, and purer mRNA), to avoid the drawbacks of the traditional methods as stated above.

EP 0 314 202 A1 discloses an improved method for messenger RNA purification which comprises either (a) treating an extract containing mRNA and ribosomal RNA with an oligo dT affinity column wherein the improvement comprises denaturing the extract prior to treating with said column or (b) treating the extract with an oligo dT affinity column and wherein the improvement comprises treating the extract with Sephadex G-100 before treating the extract with said column.

WANG ET AL., "Rapid high-yield mRNA extraction for reverse-transcription PCR", JOURNAL OF BIOCHEMICAL AND BIOPHYSICAL METHODS, AMSTERDAM, NL. vol. 70, no. 3, 8 March 2007, pages 507-509, XP005917465, ISSN: 0165-022X, DOI: 10.1016/J.JBBM.2006.10.003 discloses a method that facilitates convenient extraction of high-quality mRNA by minimizing cumbersome mechanical disruption and pipetting steps.

WO 2015/164773 A1 discloses a method of purifying mRNA including the steps of (a) precipitating mRNA from an impure preparation; (b) subjecting the impure preparation comprising precipitated mRNA to a purification process involving membrane filtration such that the precipitated mRNA is captured by a membrane; and (c) eluting the captured precipitated mRNA from the membrane by re-solubilizing the mRNA, thereby resulting in a purified mRNA solution.

FURAO LIU ET AL., "The Schlafen family: complex roles in different cell types and virus replication", CELL BIOLOGY INTERNATIONAL., Vol. 42, no. 1, 13 June 2017, pages 2-8, XP 055688375, GB ISSN: 1065-6995, DOI: 10.1002/cbin.10778 summarizes the characteristics of the Schlafen family's structures and functions.

CN 103 038 338A discloses a method for isolating target RNA from a mixed population of nucleic acids.

WO 01/25418A1 discloses methods and compositions for selectively isolating total bacterial mRNA. The general method comprises contacting a bacterial lysate comprising total bacterial mRNA and nonisolated bacterial polysomes with an exogenous enzyme under conditions wherein the enzyme selectively modifies the mRNA to form modified mRNA, and isolating the modified mRNA.

### SUMMARY OF THE INVENTION

The present disclosure is intended to overcome the defects and shortcomings of the methods for purifying total mRNA from total RNA in the prior art above, which have complicated steps, non-ideal purification effect, and difficulty in ensuring quality and integrity of mRNA. The present disclosure provides a good method for purifying total mRNA from total RNA with SLFN13. According to the present disclosure, total mRNA is purified from total RNA by using SLFN13 that specifically digests tRNA and rRNA. The method of the present disclosure not only greatly improves the purity of total mRNA, but also simplifies experimental process, saves time, and ensures the stability and integrity of total mRNA, thereby ensuring the accuracy and effectiveness of subsequent library establishment, sequencing data and other relevant experimental analysis.

Regarding the shortcomings of the two traditional purification methods above, we purify total mRNA by a novel method which introduces a specific endonuclease targeting and digests tRNA and rRNA that have the highest content in total RNA into small molecular fragments, without affecting the integrity of mRNA. This method is simple and convenient, and does not need complicated processes such as binding to a purified matrix and elution, thereby greatly reducing the degradation probability of mRNA and ensuring the purity of mRNA.

The invention is specified by the independent claim. Further embodiments are specified in the dependent claims.

The total mRNA prepared according to the present disclosure can be directly used in subsequent library establishment.

The present disclosure has the following advantages as compared to the prior art.

The present disclosure breaks the traditional concepts of RNA purification, and introduces a specific RNA endonuclease to digest and remove tRNA and rRNA from total RNA, which is simple, convenient and highly-efficient. The advantages of present disclosure can be further summarized as follows.
1) Low cost and easy availability. The purification process of the present disclosure does not need too many additional RNA purification media, such as specific RNA binding matrices, special RNA purification buffer solutions and the like. The most critical step is to purify and obtain the active endonuclease, which can be expressed by *Escherichia coli* strains, and be obtained with purity more than 90% by Ni-matrix affinity chromatography combined with size-exclusion chromatography. About 50 mg of protein (about 12 mM) can be obtained by the purification of 3 L bacteria culture, which can express the endonuclease under induction. In addition, the enzyme has high efficiency of enzyme digestion *in vitro,* and 4 pmol of the enzyme can digest 1 µg of RNA substrate in 10 to 20 min at room temperature. Therefore, the time for purifying the enzyme is short and the cost is low, however, the enzyme can be used for many times.
2) Simple and convenient operation. The use of RNA purification matrix is omitted, and the matrix balance, RNA specific-binding and elution and other processes are thus skipped. It only needs one step, adding a suitable amount of the enzyme into the RNA enzyme digestion system. In the enzyme digestion process, it just needs standing or simple rotation, without additional manual monitoring. For RNA fragments produced by enzyme digestion, they can omit a purification step, because these fragments with very small sizes do not produce too much interference to the library establishment of mRNA with larger molecular weight. The whole process is easy to be mastered and is not easy to introduce errors, and can be operated quickly and skillfully even by a beginner.
3) Time saving. Since the operation steps and experimental processes are simple, it takes less time, which can shorten the experimental period and help to ensure the stability of the RNA samples.

Even if the digested fragments are to be removed, the method can be finished within half an hour in the combination with a small RNA extraction kit. 4) Good purification effect. The tRNA and rRNA can be selectively removed in one step to ensure the purity and integrity of total mRNA. The present disclosure mainly relies on the specific endonuclease to digest and remove unnecessary RNA components. Due to the selectivity and specificity of the endonuclease digestion, tRNA and rRNA with the highest content in total RNA can be digested and removed at one time, which is more thorough than other purification methods. Since the enzyme has no digestion activity for single-stranded RNA, the integrity of mRNA can be ensured as much as possible.

5) Contributing to ensure the stability of mRNA. RNA is easy to be degraded in air. In the method of the present disclosure, it can greatly reduce the degradation probability of mRNA introduced in the experimental operation process, which is beneficial to ensure the quality of purified total mRNA, since the time-consuming steps such as sample loading and elution have been omitted.

### BRIEF DESCRIPTION OF THE DRAWINGS

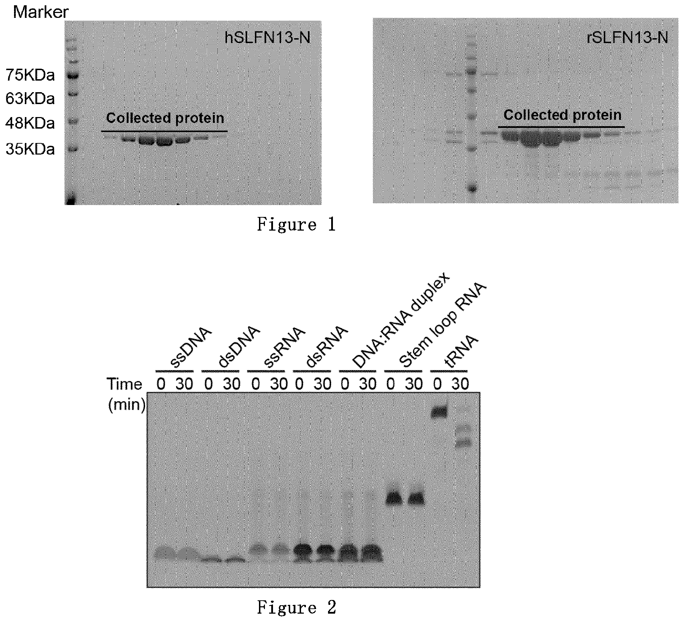

Fig. 1 is a diagram illustrating the SDS-PAGE analysis results of the samples prepared from the collection tubes corresponding to the elution peaks of monomeric protein, after the purification of hSLFN13-N and rSLFN13-N.
Fig. 2 illustrates the urea-gel electrophoresis analysis results after the selective digestion of tRNA with SLFN13-N.

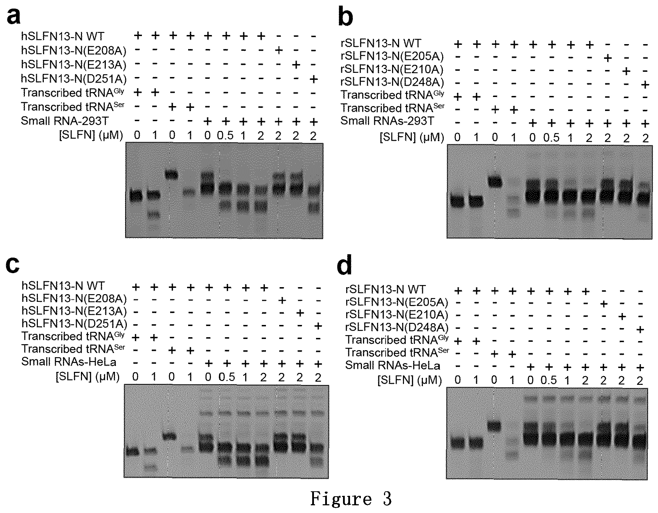

Fig. 3 illustrates the determination of the active sites of SLFN13-N and the detection results of the digestion activity for mature tRNA *in vivo.* Fig. 3a and 3b respectively illustrate the enzyme digestion effects of hSLFN13, rSLFN13 and related mutants thereof for small RNA extracted from 293T cells. Figs. 3c and 3d respectively illustrate enzyme digestion effects of hSLFN13, rSLFN13 and the related mutants thereof for small RNA extracted from HeLa cells. The small RNA mainly contains tRNA, and 5S and 5.8S rRNA.

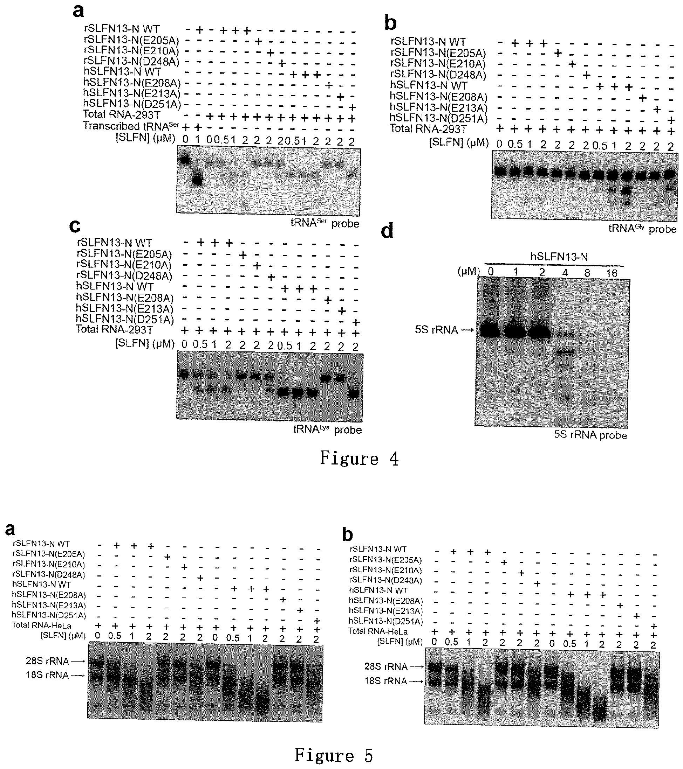

Fig. 4 illustrates the Northern blot results for verifying the enzyme digestion activity of SLFN13-N for tRNA and rRNA in total RNA extracted *in vivo.* Fig. 4a to 4c respectively illustrate the digestion results of SLFN13 for tRNA^{Ser}, tRNA^{Gly} and tRNA^{Lys}, which are detected by specific probes targeting these three mature tRNA. Fig. 4d illustrates the digestion results of SLFN13 for 5S rRNA, which are detected by a probe targeting 5S rRNA.
Fig. 5 illustrates the digestion results of SLFN13-N for rRNA in the total RNA extracted from the cells. Fig. 5a illustrates the digestion results of SLFN13 (hSLFN13 and rSLFN13) and the related mutants thereof for the total RNA extracted from 293T cells. Fig. 5b illustrates the digestion results of SLFN13 (hSLFN13 and rSLFN13) and the related mutants thereof for the total RNA extracted from HeLa cells.

### DETAILED DESCRIPTION OF THE EMBODIMENTS

Hereinafter, the present disclosure will be further described in details with reference to the embodiments, but the embodiments of the present disclosure are not limited to this.

### Example 1. Expression and purification of SLFN13

The N-terminal domains of SLFN13 (the amino acids sequence 1-355 of human SLFN13, hSLFN13-N, and the amino acids sequence 1-353 of rat SLFN13, rSLFN13-N, collectively called SLFN13-N) were individually inserted into a pET28 vector. After verification by Sanger DNA sequencing, the recombinant plasmids were transformed into a *Rossetta* (DE3) expression strain. Bacterial monocolonies can be applied into 100 ml LB medium co-supplied with kanamycin and chloramphenicol for preculture. After 12 to 16 hours, the bacteria culture was transferred, in a ratio of 1:100, into 5 L TB medium co-supplied with kanamycin and chloramphenicol to expand at 37°C. It was cooled to 17°C when OD reached 0.4 to 0.6, and 80 µM IPTG was added to induce the expression of SLFN13-N protein. After induced expression at 17°C for 16 h to 20 h, the bacteria culture was centrifuged to collect and lyse the bacteria to release the proteins. Then, a 6 × His-tag at the N-terminal of SLFN13-N was used for affinity purification with a Ni-matrix. Finally, the homogeneous protein components were separated by size-exclusion chromatography, concentrated to about 2 ng/µl (50 µM) for later use, and frozen at -80°C for storage. The purification results of hSLFN13-N and rSLFN13-N are shown in Fig. 1. It can be seen from Fig. 1 that the purity of both the proteins are more than 90%.

### Example 2

The present disclosure provides a method for purifying total mRNA from total RNA by using SLFN13, which comprises the following specific steps of:
(1) Extracting total RNA: extracting complete total RNA by using a traditional TRIzol-chloroform method for cell or tissue samples (if the samples are special, other applicable methods can be considered, provided that the quality and integrity of the total RNA can be ensured as far as possible).
(2) Performing enzyme digestion on tRNA and rRNA: calculating the amount of SLFN13 endonuclease to be added and the digestion time, according to the amount of total RNA extracted from the sample, and an approximate content ratio of tRNA, rRNA and mRNA therein. Taking contents of tRNA, rRNA and mRNA in the total RNA being respectively 12%, 83% and 3% as an example, if 10 µl total RNA (1 µg/ul) was taken, 1 µl SLFN13 (50 µM), 2 µl 10 × enzyme digestion buffer, and 7 µl ddH₂O were added to obtain 20 µl enzyme digestion system (that is, 1 µg total RNA is enzymatically digested with 5 pmol SLFN13, and the enzyme is provided in a concentration of 50 µM). The 10 × enzyme digestion buffer comprised 400 mM Tris-HCl (pH 8.0), 200 mM KCl, 40 mM MgCl₂ and 20 mM DTT. The digestion system was incubated at a room temperature for 30 min. It was demonstrated by Figs. 2 to 5 that tRNA and rRNA in the total RNA are specifically digested by SLFN13-N to fragments within 100 nt. If there are differences in the components due to the different sources of the total RNA, the usage amount of the enzyme can be appropriately increased or decreased, and is recommended to fluctuate within a range of 30%.
(3) After the enzyme digestion, directly heating at 70°C for 15 min to inactivate SLFN13-N.
(4) If there were stricter requirements for the purity of mRNA, total mRNA with higher purity was obtained for subsequent library establishment, by removing the digested tRNA and rRNA fragments in combination with a corresponding small RNA purification kit, after the step (3).

Fig. 2 illustrates the selective digestion results of SLFN13-N for tRNA. As shown by the figure, SLFN13-N was incubated with different types of nucleic acid substrates *in vitro* for enzyme digestion of 30 min and then the urea gel electrophoresis analysis was performed, and the results show that only tRNA is specifically digested by SLFN13-N.

Fig. 3 illustrates the determination of the active sites of SLFN13-N and the digestion activity thereof for mature tRNA *in vivo.* In order to better understand the digestion property of SLFN13-N, we determined the active sites of the enzyme digestion and verified the digestion activity of related mutant proteins for mature tRNA extracted from cells. We extracted small RNA with more than 90% tRNA content from HEK-293T cells and HeLa cells as substrates for the verification of enzyme digestion. The results show that SLFN13-N has similar digestion activity and property for tRNA extracted from the cells and tRNA transcribed *in vitro.*

Fig. 4 illustrates the Northern blotting results for verifying the enzyme digestion activity of SLFN13-N for tRNA and rRNA in the total RNA extracted *in vivo.* The probes targeting tRNA^{Ser}, tRNA^{Gly}, tRNA^{Lys} and 5S rRNA were designed respectively, and were labeled with P32 at 5' end thereof. The total RNA extracted from the cells was incubated and reacted with SLFN13-N, and then separated on a gel and transferred to a membrane. The corresponding probes were respectively hybridized with the products of the enzyme digestion. The results show that SLFN13-N has obvious enzyme digestion activity for tRNA and 5S rRNA in the total RNA extracted from the cells, and with the increase of the time and the amount of the enzyme, 5S rRNA can be enzymatically digested into fragments.

Fig. 5 illustrates the enzyme digestion activity of SLFN13-N for rRNA in the total RNA extracted from the cells. SLFN13-N and related enzymatically active mutants were respectively incubated and reacted with the total RNA extracted from HEK-293T cells and HeLa cells. With the increase of the concentration of the enzyme, the digestion effect was significantly enhanced, and with the increase of the time, rRNA was gradually digested into fragments.

The present disclosure breaks the traditional concepts of RNA purification, and introduces a specific RNA endonuclease to digest and remove tRNA and rRNA molecules from the total RNA, which is simple, convenient and highly-efficient. The advantages of the present disclosure can be summarized as follows.
1) Low cost and easy availability. The purification process of the present disclosure do not need too many additional RNA purification media, such as specific RNA binding matrices, special RNA purification buffer solutions and the like. The most critical step is to purify and obtain the active endonuclease, which can be expressed by *Escherichia coli* strains, and be obtained with a purity more than 90% by Ni-matrix affinity chromatography combined with size-exclusion chromatography. About 50 mg of proteins (about 12 mM) can be obtained by the purification of 3 L bacteria culture, which can express the endonuclease under induction. In addition, the enzyme has high efficiency of enzyme digestion *in vitro,* and 4 pmol of the enzyme can digest 1 µg of RNA substrate in 10 to 20 min at room temperature. Therefore, the time for purifying the enzyme once is short and the cost is low, however, the enzyme can be used for many times.
2) Simple and convenient operation. The use of RNA purification matrix is omitted, and the matrix balance, RNA specific-binding and elution and other processes are thus skipped. It only needs one step, adding a suitable amount of the enzyme into the RNA enzyme digestion system. In the enzyme digestion process, it just needs standing or simple rotation, without additional manual monitoring. For RNA fragments produced by enzyme digestion, they can omit a purification step, because these fragments with very small sizes do not produce too much interference to the library establishment of mRNA with larger molecular weight. The whole process is easy to be mastered and is not easy to introduce errors, and can be operated quickly and skillfully even by a beginner.
3) Time saving. Since the operation steps and experimental processes are simple, it takes less time, which can shorten the experimental period and help to ensure the stability of the RNA samples. Even if the digested fragments are to be removed, the method can be finished within half an hour in the combination with a small RNA extraction kit.
4) Good purification effect. The tRNA and rRNA can be selectively removed in one step to ensure the purity and integrity of total mRNA. The present disclosure mainly relies on the specific endonuclease to digest and remove unnecessary RNA components. Due to the selectivity and specificity of the endonuclease digestion, tRNA and rRNA with the highest content in the total RNA can be digested and removed at one time, which is more thorough than other purification methods. Since the enzyme has no enzyme digestion activity for single-stranded RNA, the integrity of mRNA can be ensured as much as possible.
5) Contributing to ensure the stability of mRNA. RNA is easy to be degraded in air. In the method of the present disclosure, it can greatly reduce the degradation probability of mRNA introduced in the experimental operation process, which is beneficial to ensure the quality of purified total mRNA, since the time-consuming steps such as sample loading and elution have been omitted.

## Claims

1. A method for purifying total mRNA from total RNA with SLFN13, comprising the following steps of:
(1) extracting total RNA: extracting complete total RNA from a sample by using a traditional TRIzol-chloroform method;
(2) performing enzyme digestion on tRNA and rRNA: taking purification of 10 µg total RNA as an example, adding 1 µl of 50 µM SLFN13 into 10 µl total RNA of 1 µg/µl, then adding 2 µl 10 × enzyme digestion buffer comprising 400 mM of Tris-HCl (pH 8.0), 200 mM of KCl, 40 mM of MgCl₂ and 20 mM of DTT, adding 7 µl of ddH₂O to obtain 20 µl of an enzyme digestion system; and incubating the enzyme digestion system at room temperature for 30 min; and
(3) after the enzyme digestion, directly heating at 70°C for 15 min to inactivate SLFN13, so as to obtain purified total mRNA.

2. The method for purifying total mRNA from total RNA with SLFN13 according to claim 1, wherein the sample in step (1) is one of a cell sample or a tissue sample.

3. The method for purifying total mRNA from total RNA with SLFN13 according to claim 1, wherein the SLFN13 in step (2) is one of full-length SLFN13 or an N-terminal structural domain of SLFN13, wherein the N-terminal structural domain of SLFN13 is a truncated version of human SLFN13 (Gene ID in NCBI: 146857) including amino acid residues 1-355, or a truncated version of rat SLFN13 (Gene ID in NCBI: 303378) including amino acid residues 1-353.

4. The method for purifying total mRNA from total RNA with SLFN13 according to claim 3, wherein the N-terminal structural domain of SLFN13 is prepared by an expressing and purifying method comprising:
constructing the N-terminal structural domain of SLFN13 into a pET28 vector;
after demonstrating the N-terminal structural domain with sequencing, transforming plasmids into a Rossetta (DE3) expression strain;
selecting monocolonies to preculture in 100 ml of LB medium added with double-antibiotics, kanamycin and ampicillin;
after 12 h to 16 h, transferring a bacteria solution, in a ratio of 1:100, into 5 L of TB medium added with the double- antibiotics to expand at 37°C;
cooling to 17°C when OD reaches 0.4 to 0.6;
adding 80 µM of IPTG to induce expression of SLFN13-N protein;
after induced expression at low-temperature for 16 h to 20 h, centrifuging the bacteria solution to collect and lyse the bacteria to release proteins;
performing affinity purification with a Ni-matrix by means of a 6 × His-tag at an N-terminal of SLFN13-N; and
finally separating a homogeneous protein component by size-exclusion chromatography, and concentrating to about 2 µg/µl for later use, and freezing at -80°C for storage.

## Patentansprüche

1. Verfahren zum Reinigen von Gesamt-mRNA aus Gesamt-RNA mit SLFN13, umfassend die folgenden Schritte:
1) Extrahieren von Gesamt-RNA: Extrahieren von vollständiger Gesamt-RNA aus einer Probe durch Anwenden eines herkömmlichen TRIzol-Chloroform-Verfahrens;
2) Durchführen einer Enzymverdauung auf tRNA und rRNA: bei der Reinigung von beispielsweise 10 µg Gesamt-RNA Zusetzen von 1 µl 50 µM SLFN13 zu 10 µl Gesamt-RNA mit 1 µg/µl, dann Zusetzen von 2 µl 10 × Enzymverdauungspuffer, umfassend 400 mM Tris-HCl (pH 8,0), 200 mM KCl, 40 mM MgCl₂ und 20 mM DTT, Zusetzen von 7 µl ddH₂O, um 20 µl eines Enzymverdauungssystems zu erhalten; und Inkubieren des Enzymverdauungssystems bei Raumtemperatur für 30 Minuten; und
3) nach der Enzymverdauung direktes Erwärmen bei 70 °C für 15 Minuten, um SLFN13 zu inaktivieren, um gereinigte Gesamt-mRNa zu erhalten.

2. Verfahren zum Reinigen von Gesamt-mRNA aus Gesamt-RNA mit SLFN13 nach Anspruch 1, wobei die Probe in Schritt (1) eine einer Zellprobe oder einer Gewebeprobe ist.

3. Verfahren zum Reinigen von Gesamt-mRNA aus Gesamt-RNA mit SLFN13 nach Anspruch 1, wobei das SLFN13 in Schritt (2) eines eines SLFN13 voller Länge oder einer N-terminalen Strukturdomäne von SLFN13 ist, wobei die N-terminale Strukturdomäne von SLFN13 eine trunkierte Version von humanem SLFN13 (Gen-ID in NCBI: 146857), einschließlich Aminosäureresten 1-355, oder eine trunkierte Version von Ratten-SLNF13 (Gen-ID in NCBI: 303378), einschließlich Aminosäureresten 1-353, ist.

4. Verfahren zum Reinigen von Gesamt-mRNA aus Gesamt-RNA mit SLFN13 nach Anspruch 3, wobei die N-terminale Strukturdomäne von SLFN13 durch ein Expressions- und Reinigungsverfahren hergestellt wird, das Folgendes umfasst:
Konstruieren der N-terminalen Strukturdomäne von SLFN13 in einen pET28-Vektor;
nach Bestätigen der N-terminalen Strukturdomäne mittels Sequenzieren Transformieren von Plasmiden in einen Rossetta (DE3)-Expressionsstrang;
Auswählen von Monokolonien zum Vorkultivieren in 100 ml LB-Medium, versetzt mit zwei Antibiotika, Kanamycin und Ampicillin;
nach 12 h bis 16 h Übertragen einer Bakterienlösung in einem Verhältnis von 1 : 100 in 5 I TB-Medium, versetzt mit den zwei Antibiotika, um bei 37 °C zu expandieren;
Abkühlen auf 17 °C, wenn die OD 0,4 bis 0.6 erreicht;
Zusetzen von 80 µM IPTG, um die Expression des SLFN13-Proteins zu induzieren;
nach der induzierten Expression bei niedriger Temperatur für 16 h bis 20 h Zentrifugieren der Bakterienlösung, um die Bakterien zu sammeln und zu lysieren, um Proteine freizusetzen;
Durchführen einer Affinitätsreinigung mit einer Ni-Matrix anhand eines 6 × His-Tags an einem N-Terminus von SLFN13-N; und
abschließendes Abtrennen einer homogenen Proteinkomponente durch Größenausschlusschromatografie und Konzentrieren auf etwa 2 µg/µl zur späteren Verwendung und Einfrieren bei -80 °C zur Lagerung.

## Revendications

1. Procédé de purification d'ARNm total à partir d'ARN total avec SLFN13 comprenant les étapes suivantes :
1) extraction d'ARN total : extraction de la totalité de l'ARN total d'un échantillon en utilisant un procédé classique au TRIzol-chloroforme ;
2) réalisation d'une digestion enzymatique sur l'ARNt et l'ARNr : prendre la purification de 10 µg d'ARN total par exemple, ajouter 1 µl de SLFN13 50 µM dans 10 µl d'ARN de µg/µl, puis ajouter 2 µl de tampon de digestion enzymatique 10 × comprenant 400 mM de Tris-HCl (pH 8,0), 200 mM de KCl, 40 mM de MgCl₂ et 20 mM de DTT, ajouter 7 pl de H₂O bidistillée pour obtenir 20 µl de système de digestion enzymatique ; et mettre à incuber le système de digestion enzymatique à température ambiante pendant 30 minutes ; et
3) après la digestion enzymatique, chauffage direct à 70 °C pendant 15 minutes pour inactiver le SLFN13, pour obtenir de l'ARNm total purifié.

2. Procédé de purification d'ARNm total à partir d'ARN total avec SLFN13 selon la revendication 1, dans lequel l'échantillon à l'étape (1) est l'un d'un échantillon cellulaire ou d'un échantillon tissulaire.

3. Procédé de purification d'ARNm total à partir d'ARN total avec SLFN13 selon la revendication 1, dans lequel le SLFN13 à l'étape (2) est l'un de SLFN13 de longueur totale ou d'un domaine structural N-terminal de SLFN13, dans lequel le domaine structural N-terminal de SLFN13 est une version tronquée du SLFN13 humain (ID du gène dans le NCBI : 146857) comprenant les résidus d'acides aminés 1 à 355 ou une version tronquée de SLFN13 de rat (ID du gène dans le NCBI : 303378) incluant les résidus d'acides aminés 1 à 353.

4. Procédé de purification d'ARNm total à partir d'ARN total avec SLFN13 selon la revendication 3, dans lequel le domaine structural N-terminal de SLFN13 est préparé par un procédé d'expression et de purification comprenant :
la construction du domaine structural N-terminal de SLFN13 dans un vecteur pET28 ;
après la démonstration du domaine structural N-terminal par séquençage, la transformation de plasmides dans une souche d'expression de Rossetta (DE3) ;
la sélection de monocolonies pour préculture dans 100 ml de milieu LB additionné de deux antibiotiques, kanamycine et ampicilline ;
après 12 h à 16 h, le transfert d'une solution de bactéries, en un rapport de 1:100, dans 5 I de milieu TB aditionné des deux antibiotiques pour une expansion à 37 °C ;
le refroidissement à 17 °C lorsque la DO atteint 0,4 à 0,6 ;
l'ajout de 80 µM d'IPTG pour induire l'expression de la protéine SLFN13-N ;
après l'expression induite à basse température pendant 16 h à 20 h, la centrifugation de la solution de bactéries pour collecter et lyser les bactéries pour libérer les protéines ;
la réalisation d'une purification par affinité avec une matrice de Ni au moyen d'un His-tag 6 × et d'une extrémité N-terminale de SLFN13-N ; et
finalement la séparation d'un composant protéique homogène par chromatographie d'exclusion diffusion, et concentration à environ 2 µg/µl pour une utilisation ultérieure, et la congélation à -80 °C pour stockage.
